(19) Europäisches Patentamt
European Patent Office
Office européen des brevets

(11) **EP 4 411 598 A1**

(12) **EUROPEAN PATENT APPLICATION**

(43) Date of publication:
**07.08.2024 Bulletin 2024/32**

(21) Application number: **24152568.2**

(22) Date of filing: **18.01.2024**

(51) International Patent Classification (IPC):
**G06N 7/01** *(2023.01)*   **G16B 5/20** *(2019.01)*

(52) Cooperative Patent Classification (CPC):
**G06N 7/01; G16B 5/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.01.2023 JP 2023012525**

(71) Applicants:
• **FUJITSU LIMITED**
  **Kawasaki-shi, Kanagawa 211-8588 (JP)**
• **RIKEN**
  **Wako-shi, Saitama 351-0198 (JP)**

(72) Inventors:
• **YAMAZAKI, Kimihiro**
  **Kawasaki-shi, Kanagawa, 211-8588 (JP)**
• **WADA, Yuichiro**
  **Kawasaki-shi, Kanagawa, 211-8588 (JP)**
• **WADA, Mutsuyo**
  **Kawasaki-shi, Kanagawa, 211-8588 (JP)**
• **KATOH, Takashi**
  **Kawasaki-shi, Kanagawa, 211-8588 (JP)**
• **TOKUHISA, Atsushi**
  **Wako-shi, Saitama, 351-0198 (JP)**

(74) Representative: **Hoffmann Eitle**
**Patent- und Rechtsanwälte PartmbB**
**Arabellastraße 30**
**81925 München (DE)**

(54) **INFORMATION OUTPUT PROGRAM, INFORMATION OUTPUT METHOD, AND INFORMATION OUTPUT DEVICE**

(57)     An information output program causes a computer to execute a process including: acquiring(S100) a first point and a second point in a presence probability distribution of a state of an object; specifying(S102) a plurality of points serving as candidates for a transition destination from the first point; selecting(S102) a third point from the points based on a distance from the first point to each of the points and probability during transition; and outputting(S103) a transition path from the first point to the second point including the third point as state transition information on the object.

FIG.1

**Description**

[Technical Field]

The embodiments discussed herein are related to an information output program, an information output method, and an information output device.

[Background Art]

Analyzing state transition of molecules, such as proteins, can contribute to drug discovery and other applications. While it is difficult to directly observe the state transition of molecules through experiments or the like, one specific scene can be collected by various experimental techniques. The information on the scene collected in this manner can be used to estimate the presence probability distribution and the shape of the molecule.

For example, conventional technologies have been developed to model the presence probability distribution of a molecule by Gaussian mixture models (GMMs) with two classes and to construct a probable pathways between mean vectors of the GMMs with two classes based on mathematical definition of a point set that gives the maximum value to the GMMs. The related technologies are described, for example, in: Non-Patent document 1: S. Ray and B. Lindsay: The topography of multivariate normal mixtures. Annals of Statistics 33 2042-2065 (2005), and Non-Patent document 2: Hennig, C.: Ridgeline plot and clusterwise stability as tools for merging Gaussian mixture components. In Classification as a Tool for Research (pp. 109-116) (2010).

[Summary of invention]

[Technical Problem]

The conventional technologies described above, however, have difficulty in constructing the most probable transition pathways in a case where the number of classes of the GMMs is not 2. For example, in many cases useful for drug discovery and other applications, the presence probability distribution of a molecule is represented by the GMMs the number of classes of which is 3 or more. When the number of classes of the GMMs is 3 or more, the number of maximum values is not necessarily equal to the number of mean vectors. This is because another maximum value other than the maximum values corresponding to the mean vectors can be generated in the GMMs due to the effect of superposition of the maximum value corresponding to the mean vector with the maximum value corresponding to the mean vector. As a result, the most probable transition pathways constructed by the conventional technologies described above has errors because the effect of superposition of the maximum values corresponding to the mean vectors is ignored.

Accordingly, it is an object in one aspect of an embodiment of the present invention to provide an information output program, an information output method, and an information output device that can output the most probable transition pathways.

[Solution to Problem]

According to an aspect of an embodiment, an information output program causes a computer to execute a process including: acquiring a first point and a second point in a presence probability distribution of a state of an object; specifying a plurality of points serving as candidates for a transition destination from the first point; selecting a third point from the points based on a distance from the first point to each of the points and probability during transition; and outputting a transition path from the first point to the second point including the third point as state transition information on the object.

[Advantageous Effects of Invention]

It is possible to output the most probable transition pathways.

[Brief Description of Drawings]

FIG. 1 is a block diagram of an example of a functional configuration of a server device;
FIG. 2 is a schematic for explaining path generation;
FIG. 3 is a flowchart of an information output process;

FIG. 4 is a diagram of an example of path generation results; and
FIG. 5 is a diagram of an example of a hardware configuration.

[Embodiments for Carrying Out the Invention]

Preferred embodiments will be explained with reference to accompanying drawings. Each embodiment only indicates one example or aspect, and the values, the ranges of functions, and the use scenes are not limited by the embodiments. The embodiments can be appropriately combined without contradicting the processing contents.

First Embodiment

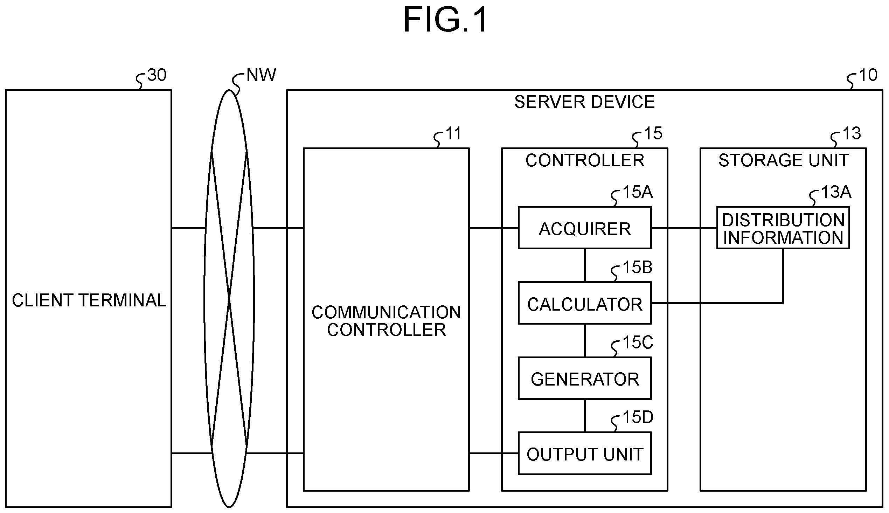

FIG. 1 is a block diagram of an example of a functional configuration of a server device 10. The server device 10 illustrated in FIG. 1 implements an information output function of receiving GMMs in which the presence probability distribution of a molecule is modeled and a pair of any two points on the GMMs as an input and calculating and outputting a probable pathway between the pair of two points. The "probable pathways" herein is also called a ridge (ridgeline). The "probable pathways" is a path having a shorter path length and a larger sum of probability densities on the path than other paths, for example. The path length is not necessarily the shortest path length, and the sum of probability densities on the path is not necessarily the largest sum.
While the following describes an example where the presence probability distribution of a molecule, such as a protein, is modeled by GMMs, the object is not limited to molecules. Examples of objects other than molecules include, but are not limited to, networks with the concept of energy (e.g., social networks that can express nodes corresponding to accounts by a blowing-up degree indicating the degree of concentration of criticism or the like).
The server device 10 is an example of a computer that implements the information output function described above. For example, the server device 10 can implement the information output function described above as a cloud service by providing it as a platform as a service (PaaS) or software as a service (SaaS) application. In addition, the server device 10 can be provided as a server that implements the information output function described above on-premises.
As illustrated in FIG. 1, the server device 10 can be communicably connected to a client terminal 30 via a network NW. The network NW may be, for example, any type of wired or wireless communication network, such as the Internet and a local area network (LAN). While FIG. 1 illustrates an example where one client terminal 30 is connected to one server device 10, any number of client terminals 30 may be connected.
The client terminal 30 corresponds to an example of a computer supplied with the information output function described above. The client terminal 30 may be provided by a desktop or laptop personal computer, for example. This is given by way of example only, and the client terminal 30 may be any computer, such as a portable terminal device or a wearable terminal.
While FIG. 1 illustrates an example where the information output function described above is provided by a client-server system, this is given by way of example only. The information output function described above may be provided stand-alone.
The following describes an example of the functional configuration of the server device 10 according to the present embodiment. FIG. 1 schematically illustrates the blocks relating to the information output function of the server device 10. As illustrated in FIG. 1, the server device 10 includes a communication controller 11, a storage unit 13, and a controller 15. FIG. 1 only extracts and illustrates functional units relating to the information output function described above, and functional units other than those illustrated in FIG. 1 may be provided to the server device 10.
The communication controller 11 is a functional unit that controls communications with other devices, such as the client terminal 30. For example, the communication controller 11 can be provided by a network interface card, such as a LAN card. In one aspect, the communication controller 11 receives a request from the client terminal 30 to output information on probable pathways between any two points on the GMMs or outputs a response to the request to the client terminal 30.
The storage unit 13 is a functional unit that stores therein various data. For example, the storage unit 13 is provided by an internal, external, or auxiliary storage of the server device 10. The storage unit 13 stores therein distribution information 13A, for example. The distribution information 13A will be described later in the description of registration or reference.
The controller 15 is a functional unit that collectively controls the server device 10. The controller 15 can be provided by a hardware processor, for example. As illustrated in FIG. 1, the controller 15 includes an acquirer 15A, a calculator 15B, a generator 15C, and an output unit 15D. The controller 15 may be provided by hardwired logic or the like.
The acquirer 15A is a processing unit that acquires GMMs in which the presence probability distribution of a molecule is modeled and a pair of any two points on the GMMs as an input. For example, when the acquirer 15A receives a request from the client terminal 30 to output information on probable pathways between any two points on the GMMs, it can receive specification of the GMMs and the pair of two points on the GMMs.

For example, the acquirer 15A can acquire $P_\psi(z)$ in which the presence probability distribution of the molecule is modeled by receiving specification of parameters $\pi_c$, $\mu_c$, and $\Sigma_c$ that define the GMMs or what is called mixture Gaussian distribution $P_\psi(z)$ expressed by Expression (1) below. Note that $\psi$, $\pi_c$, $\mu_c$, and $\Sigma_c$ herein are provided with a hat. The number C of Gaussian distributions N specified in this manner, that is, the number of classes of the GMMs may be 3 or more. The acquirer 15A can also acquire a pair of two points $(z^{(0)}, z^{(1)})$ by receiving specification of any two points on $P_\psi(z)$, that is, two points for which a probable pathway is to be defined. Neither of the two specified points is not necessarily a mean vector. The Gaussian mixture distribution $P_\psi(z)$ and the pair of two points $(z^{(0)}, z^{(1)})$ acquired in this manner may be stored in the storage unit 13 as the distribution information 13A.

$$P_{\hat\psi}(z) = \Sigma_{c=1}^C \hat\pi_c N\left(z; \hat\mu_c, \hat\Sigma_c\right) \qquad \cdots (1)$$

The calculator 15B is a processing unit that calculates the class to which each of the two points specified as a pair belongs. For example, the calculator 15B can calculate the class to which $z^{(0)}$ belongs according to the algorithm of Bayes' theorem described in Reference 1 below, that is, $c_0 = \text{argmax}_{c=1, \ldots, C} P_\psi(C|z^{(0)}) = \pi_c N_c(z^{(0)}; \mu_c, \Sigma_c)/P_\psi(z^{(0)})$. Similarly to $z^{(0)}$, the calculator 15B can also calculate the class to which $z^{(1)}$ belongs. In the following description, the mean vector corresponding to the class to which $z^{(0)}$ belongs is denoted as $\mu_i$, and the mean vector corresponding to the class to which $z^{(1)}$ belongs is denoted as $\mu_j$. Note that $\mu_i$ and $\mu_j$ herein are also provided with a hat.

Reference 1: James Joyce. Bayes' theorem. The Stanford Encyclopedia of Philosophy, 2003.

The generator 15C is a processing unit that generates a probable pathway between two mean vectors. For example, the generator 15C probabilistically constructs a probable pathway $z_{0=\mu_i} \to z_1 \to z_2 \to \ldots \to z_{K-1} \to z_{K=\mu_j}$ from the mean vector $\mu_i$ to the mean vector $\mu_j$ so as to satisfy the two requirements below. The first requirement is, for example, that the path length of Expression (2) below is as short as possible. The second requirement is that the average probability on the path in Expression (3) below is as large as possible.

$$\Sigma_{k=0}^K \|z_k - z_{k-1}\|_2 \qquad \cdots (2)$$

$$\Sigma_{k=0}^K \|z_k - z_{k-1}\|_2 P_{\hat\psi}(z_k) / \Sigma_{k=0}^K \|z_k - z_{k-1}\|_2 \qquad \cdots (3)$$

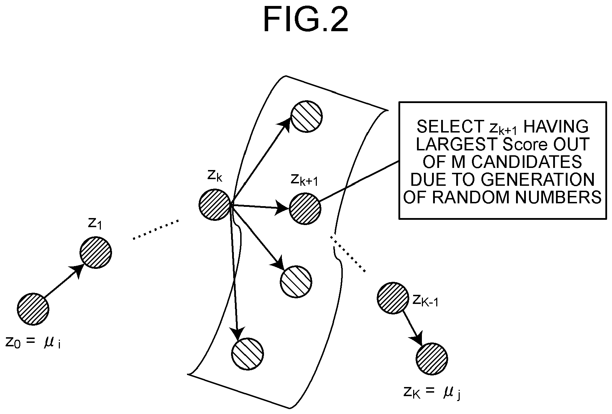

More specifically, the probable pathways $z_0 \to z_1 \to z_2 \to \ldots \to z_{K-1} \to z_K$ can be inductively defined as follows. FIG. 2 is a schematic for explaining path generation. As illustrated in FIG. 2, the probable pathways $z_0$ from the mean vector $\mu_i$ to the mean vector $\mu_j$ is divided into K sections. In the following description, k refers to an index indicating an integer from 1 to K. m candidate points $(z^{(1)}_{k+1}, \ldots, z^{(m)}_{k+1})$ to which transition is made from $z_k$ in each of the K sections are probabilistically sampled according to Expression (4) below. Under Expression (4), $\alpha_c \geq 0$ and $c \neq i,j$ are randomly defined so as to satisfy $\Sigma_{c \neq i,j}\alpha_c = (1-\delta(k))\alpha_i(k)$.

$$z_{k+1}^{(l)} = \left((1 - \alpha_i(k))\Sigma_i^{-1} + \delta(k)\alpha_i(k)\Sigma_j^{-1} + \Sigma_{c \neq i,j}\alpha_c \Sigma_c^{-1}\right)^{-1}$$
$$\times \left((1 - \alpha_i(k))\Sigma_i^{-1}\mu_i + \delta(k)\alpha_i(k)\Sigma_j^{-1}\mu_j + \Sigma_{c \neq i,j}\alpha_c \Sigma_c^{-1}\mu_c\right)$$
$$\cdots (4)$$

The pair $(\alpha_i(k), \delta(k))$ is a function with k as an argument, for example, and a function can be set that satisfies $(0,0)$ for $k=0$, $(1,1)$ for $k=K$, and $0 < \alpha_i(k)$ and $\delta(k) < 1$ when k is not 0 or K. By introducing the pair $(\alpha_i(k), \delta(k))$ into the sampling of m candidate points, each of the m candidate points can be set on the path from $z_k$ to $\mu_j$.

In addition, Expression (4) incorporates the mathematical definition described in Non-Patent Literature 1 above. For example, in Non-Patent Literature 1, a function $x(\alpha) \in R^d$ with d as a dimension and $\alpha = (\alpha_1, \ldots, \alpha_c)$ (probability vector) as an argument is defined as in Expression (5) below. Note that $\mu_i$ and $\Sigma_i$ refer to the average and the variance matrix of the i-th Gaussian distribution. In this case, $x(\alpha)$ is the point that gives the maximum value to the GMMs. By introducing limitation of the maximum point described in Non-Patent Literature 1 into the sampling of the m candidate points, it is secured that each of the m candidate points is a point with maximality.

$$x(\alpha) = \left(\Sigma_{c=1}^{C} \alpha_c \Sigma_c^{-1}\right)^{-1}\left(\Sigma_{c=1}^{C} \alpha_c \Sigma_c^{-1} \mu_c\right) \quad \cdots (5)$$

After the m candidate points are sampled in this manner, the generator 15C calculates a score for each of the m candidate points. For example, the score for the l-th candidate point $z^{(1)}_{k+1}$ can be calculated according to one of the functions $s_0$ to $s_2$ expressed by Expressions (7) to (9) below with a probability density p and a distance d expressed by Expression (6) below as arguments. In Expressions (6) to (9) below, p refers to the probability density on the l-th candidate point $z^{(1)}_{k+1}$, and d refers to the distance between the point $z_k$ and the l-th candidate point $z^{(1)}_{k+1}$. Besides $s_0$ to $s_2$, $s_3$ expressed by Expression (10) below can also be used to calculate the score.

$$p = P_{\hat{\psi}}\left(z_{k+1}^{(l)}\right), d = \left\|\left(z_k, P_{\hat{\psi}}(z_k)\right) - \left(z_{k+1}^{(l)}, p\right)\right\|_2 \quad \cdots (6)$$

$$s_0 = p/d \tag{7}$$

$$s_1 = p \cdot \exp(-d^2/\text{var}) \tag{8}$$

$$s_2 = 1/d \tag{9}$$

$$s_3 = f(p) + rg(d) \tag{10}$$

$$f(p) = p$$

$$g(d) = \exp(-d^2)$$

The generator 15C sets $z^{(1)}_{k+1}$ having the largest score out of the m candidate points to $z_{k+1}$. As a result, the candidate point conforming to the two requirements described above can be selected out of the m candidate points.

The output unit 15D is a processing unit that outputs state transition information including the probable pathways for the specified pair of two points. For example, the output unit 15D defines a probable pathway from $z^{(0)}$ to $z^{(1)}$ based on the probable pathway from the mean vector $\mu_i$ to the mean vector $\mu_j$ generated by the generator 15C as follows. Specifically, the output unit 15D sets the probable pathways from $z^{(0)}$ to $z^{(1)}$ to $z^{(0)} \to z_0 \to z_1 \to z_2 \to ... \to z_{K-1} \to z_K \to z^{(1)}$. In this case, a straight line may be set for each of the paths in the sections from $z^{(0)}$ to $\mu_1(=z_0)$ and from $\mu_j(=z_K)$ to $z^{(1)}$. The output unit 15D then outputs the probable pathways from $z^{(0)}$ to $z^{(1)}$ to the client terminal 30. The output unit 15D, for example, may display the point set included in the path by plotting them on the map of the GMMs or may display the position and the probability density of the point set included in the path.

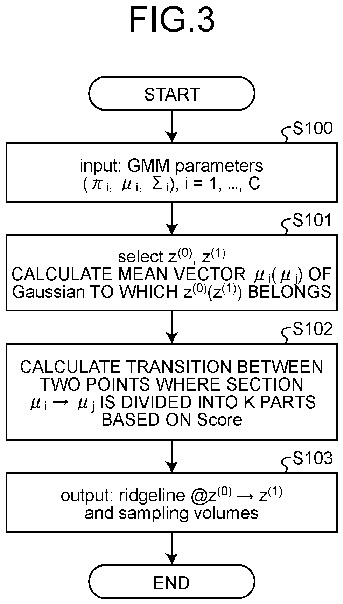

FIG. 3 is a flowchart of the information output process. As illustrated in FIG. 3, the acquirer 15A acquires the parameters $\pi_c$, $\mu_c$, and $\Sigma_c$ that define the GMMs or what is called the mixture Gaussian distribution $P_\psi(z)$ (Step S100).

Subsequently, the calculator 15B calculates the mean vector $\mu_j$ of the Gaussian distribution to which $z^{(0)}$ belongs and the mean vector $\mu_j$ of the Gaussian distribution to which $z^{(1)}$ belongs according to the algorithm of Bayes' theorem described in Reference 1 above (Step S101).

Subsequently, the generator 15C calculates the transition between the two points where the section from the mean vector $\mu_i$ to the mean vector $\mu_j$ is divided into K parts based on the score indicating the degree of satisfying the two requirements described above (Step S102).

The output unit 15D then outputs the probable pathways from $z^{(0)}$ to $z^{(1)}$ to any desired output destination, such as the client terminal 30 (Step S103), and terminates the process.

As described above, the information output function according to the present embodiment outputs the path obtained by selecting such transition that the path length is shorter and the average probability on the path is larger for each transition between two points where the section between two mean vectors on the GMMs is divided into K parts. As a result, the information output function can output a path that suppresses errors caused by superposition of the maximum values corresponding to three or more mean vectors. If the number of dimensions of the mean vectors of the GMMs is large, such as eight dimensions, robust results can be obtained. Therefore, the information output function according to the present embodiment can output the information on the most probable transition pathways. Such information output can contribute to analyzing the reaction mechanism of a molecule because it enables expressing a likely change of the molecule and its energy transition process.

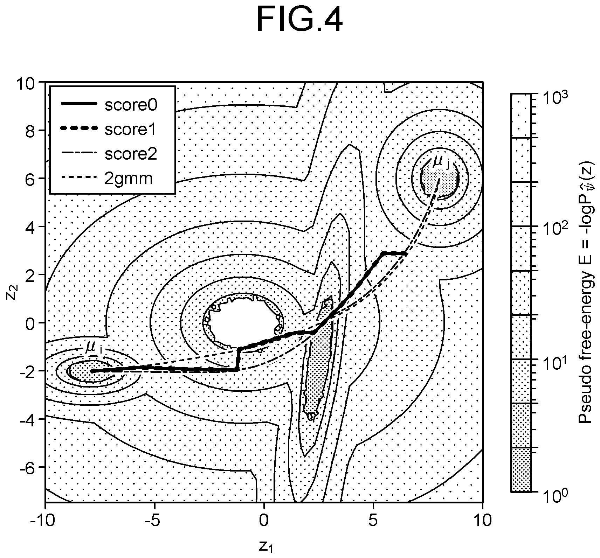

FIG. 4 is a diagram of an example of path generation results. FIG. 4 illustrates a graph obtained by mapping the GMMs with 4 classes as an example of the presence probability distribution of a molecule. The vertical and horizontal axes of the graph illustrated in FIG. 4 correspond to two-dimensional vectors including $z_1$ and $z_2$. In the graph illustrated in FIG. 4, pseudo-free energy, or $-\log(P\psi(z))$, calculated from the presence probability density of each point on the two-dimensional vector plane is drawn by hatching indicated in the legend. FIG. 4 illustrates an example where the probable pathways between the mean vector $\mu_i$ and the mean vector $\mu_j$ is generated based on the score functions $s_0$, $s_1$, and $s_2$ and the conventional technology (2 gmm). The paths for the score functions $s_0$, $s_1$, and $s_2$ and 2 gmm are plotted on the GMMs. As illustrated in FIG. 4, the conventional technology (2 gmm) generates a geometric path that ignores the peaks of the maximum values corresponding to the other mean vectors. By contrast, it is clear that any of the score functions $s_0$, $s_1$, and $s_2$ generates a path that reflects the interference of the other peaks.

Second Embodiment

While the embodiment of the device according to the present disclosure has been described, the present invention may be embodied in various different forms besides the embodiment described above. The following describes other embodiments included in the present invention.

The processing procedure, the control procedure, the specific names, and the information including various data and parameters described in the specification and drawings according to the first embodiment may be appropriately changed unless otherwise specified.

The specific forms of dispersion and integration of the components of each device are not limited to those illustrated in the figures. In other words, all or part of the components may be functionally or physically dispersed and integrated in any desired units depending on various loads and use conditions. Furthermore, all or any part of the processing functions of each device can be implemented by a CPU and a computer program analyzed and executed by the CPU or as hardware using wired logic.

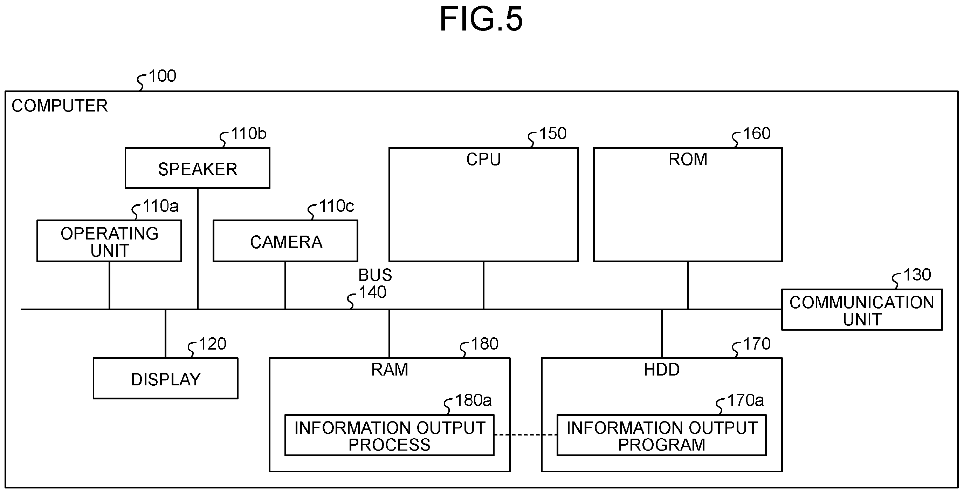

The various processing described in the first embodiment can be performed by executing a computer program prepared in advance on a computer, such as a personal computer and a workstation. The following describes an example of a computer that executes an information output program having the same functions as those according to the first embodiment and the second embodiment with reference to FIG. 5.

FIG. 5 is a diagram of an example of a hardware configuration. As illustrated in FIG. 5, a computer 100 includes an operating unit 110a, a speaker 110b, a camera 110c, a display 120, and a communication unit 130. The computer 100 also includes a CPU 150, a ROM 160, an HDD 170, and a RAM 180. These components 110 to 180 are connected via a bus 140.

As illustrated in FIG. 5, the HDD 170 stores therein an information output program 170a that implements the same functions as those of the acquirer 15A, the calculator 15B, the generator 15C, and the output unit 15D described in the first embodiment. The information output program 170a may be integrated or separated in the same manner as the components of the acquirer 15A, the calculator 15B, the generator 15C, and the output unit 15D illustrated in FIG. 1. In other words, the HDD 170 does not necessarily store therein all the data described in the first embodiment and may store therein only the data used for processing.

Under the environment described above, the CPU 150 reads the information output program 170a from the HDD 170 and loads it into the RAM 180. As a result, the information output program 170a functions as an information output process 180a as illustrated in FIG. 5. The information output process 180a loads various data read from the HDD 170 into an area allocated to the information output process 180a in the storage area of the RAM 180 and performs various processing using the loaded various data. Examples of the processing performed by the information output process 180a include the processing illustrated in FIG. 3. In the CPU 150, all the processing units described in the first embodiment do not necessarily operate, and only the processing unit corresponding to the processing to be performed may be virtually implemented.

The information output program 170a is not necessarily stored in the HDD 170 or the ROM 160 in advance. For example, the information output program 170a is stored in a "portable physical medium", such as a flexible disc or what is called an FD, a CD-ROM, a DVD disc, a magneto-optical disc, and an IC card, inserted into the computer 100. The computer 100 may retrieve and execute the information output program 170a from the portable physical medium. Alternatively, the information output program 170a is stored in another computer or server device connected to the computer 100 via a public line, the Internet, a LAN, a WAN, or the like. The information output program 170a stored in this manner may be downloaded and then executed by the computer 100.

**Claims**

1. An information output program that causes a computer to execute a process comprising:

   acquiring(S100) a first point and a second point in a presence probability distribution of a state of an object;
   specifying(S102) a plurality of points serving as candidates for a transition destination from the first point;
   selecting(S102) a third point from the points based on a distance from the first point to each of the points and probability during transition; and
   outputting(S103) a transition path from the first point to the second point including the third point as state transition information on the object.

2. The information output program according to claim 1, wherein the selecting(S102) includes calculating a score for each of the points that increases as the distance becomes shorter and increases as the average of the probability during transition becomes higher and selecting a point the score of which is largest out of the points as the third point.

3. The information output program according to claim 1, wherein the specifying(S102) includes specifying the points based on a point set that gives the maximum value to the presence probability distribution.

4. The information output program according to claim 1, wherein the presence probability distribution corresponds to a mixture Gaussian distribution.

5. The information output program according to claim 4, wherein the process further includes calculating(S101) a first mean vector to which the first point belongs out of mean vectors included in the mixture Gaussian distribution and calculating(S101) a second mean vector to which the second point belongs out of mean vectors included in the mixture Gaussian distribution, wherein

   the specifying(S102) includes specifying a plurality of points serving as candidates for a transition destination from the first mean vector,
   the selecting(S102) includes selecting a third point based on a distance from the first mean vector to each of the points and probability during transition, and
   the outputting(S103) includes outputting, as state transition information on the object, a transition path from the first point to the first mean vector, a transition path from the first mean vector to the second mean vector including the third point, and a transition path from the second mean vector to the second point.

6. An information output method carried out by a computer(100) comprising:

   acquiring(S100) a first point and a second point in a presence probability distribution of a state of an object;
   specifying(S102) a plurality of points serving as candidates for a transition destination from the first point;
   selecting(S102) a third point from the points based on a distance from the first point to each of the points and probability during transition; and
   outputting(S103) a transition path from the first point to the second point including the third point as state transition information on the object.

7. The information output method according to claim 6, wherein the selecting(S102) includes calculating a score for each of the points that increases as the distance becomes shorter and increases as the average of the probability during transition becomes higher and selecting a point the score of which is largest out of the points as the third point.

8. The information output method according to claim 6, wherein the specifying(S102) includes specifying the points based on a point set that gives the maximum value to the presence probability distribution.

9. The information output method according to claim 6, wherein the presence probability distribution corresponds to a mixture Gaussian distribution.

10. The information output method according to claim 9, further including calculating(S101) a first mean vector to which the first point belongs out of mean vectors included in the mixture Gaussian distribution and calculating(S101) a second mean vector to which the second point belongs out of mean vectors included in the mixture Gaussian distribution, wherein

the specifying(S102) includes specifying a plurality of points serving as candidates for a transition destination from the first mean vector,

the selecting(S102) includes selecting a third point based on a distance from the first mean vector to each of the points and probability during transition, and

the outputting(S103) includes outputting, as state transition information on the object, a transition path from the first point to the first mean vector, a transition path from the first mean vector to the second mean vector including the third point, and a transition path from the second mean vector to the second point.

11. An information output device(10) comprising:

an acquiring unit(15A) configured to acquire a first point and a second point in a presence probability distribution of a state of an object;

a specifying unit(15C) configured to specify a plurality of points serving as candidates for a transition destination from the first point;

a selecting unit(15C) configured to select a third point from the points based on a distance from the first point to each of the points and probability during transition; and

an outputting unit(15D) configured to output a transition path from the first point to the second point including the third point as state transition information on the object.

12. The information output device according to claim 11, wherein the selecting unit(15C) calculates a score for each of the points that increases as the distance becomes shorter and increases as the average of the probability during transition becomes higher and selects a point the score of which is largest out of the points as the third point.

13. The information output device according to claim 11, wherein the specifying unit(15C) specifies the points based on a point set that gives the maximum value to the presence probability distribution.

14. The information output device according to claim 11, wherein the presence probability distribution corresponds to a mixture Gaussian distribution.

15. The information output device according to claim 14, further including a calculating unit(15B) configured to calculate a first mean vector to which the first point belongs out of mean vectors included in the mixture Gaussian distribution and calculate a second mean vector to which the second point belongs out of mean vectors included in the mixture Gaussian distribution, wherein

the specifying unit(15C) specifies a plurality of points serving as candidates for a transition destination from the first mean vector,

the selecting unit(15C) selects a third point based on a distance from the first mean vector to each of the points and probability during transition, and

the outputting unit(15D) outputs a transition path from the first point to the first mean vector, a transition path from the first mean vector to the second mean vector including the third point, and a transition path from the second mean vector to the second point as state transition information on the object.

# FIG.1

EP 4 411 598 A1

# FIG.2

SELECT $z_{k+1}$ HAVING LARGEST Score OUT OF M CANDIDATES DUE TO GENERATION OF RANDOM NUMBERS

$z_k$

$z_{k+1}$

$z_1$

$z_0 = \mu_i$

$z_{K-1}$

$z_K = \mu_j$

# FIG.3

START

S100

input: GMM parameters
$(\pi_i, \mu_i, \Sigma_i)$, i = 1, ..., C

S101

select $z^{(0)}$, $z^{(1)}$
CALCULATE MEAN VECTOR $\mu_i(\mu_j)$ OF
Gaussian TO WHICH $z^{(0)}(z^{(1)})$ BELONGS

S102

CALCULATE TRANSITION BETWEEN
TWO POINTS WHERE SECTION
$\mu_i \rightarrow \mu_j$ IS DIVIDED INTO K PARTS
BASED ON Score

S103

output: ridgeline $@z^{(0)} \rightarrow z^{(1)}$
and sampling volumes

END

# FIG.4

# FIG.5

EP 4 411 598 A1

**EP 4 411 598 A1**

Europäisches Patentamt
European Patent Office
Office européen des brevets

# EUROPEAN SEARCH REPORT

Application Number

EP 24 15 2568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MILAD R VAHID ET AL: "Fisher information matrix for single molecules with stochastic trajectories", ARXIV.ORG, CORNELL UNIVERSITY LIBRARY, 201 OLIN LIBRARY CORNELL UNIVERSITY ITHACA, NY 14853, 7 August 2018 (2018-08-07), XP081607881, DOI: 10.1137/19M1242562 * abstract; figures 2,5 * * 1. Introduction * * 2. Fundamental data model * * 4. Maximum likelihood estimation * | 1-15 | INV. G06N7/01 G16B5/20 |
| A | HAN R ET AL: "Trajectory-based machine learning method and its application to molecular dynamics", MOLECULAR PHYSICS, vol. 118, no. 19-20, 17 October 2020 (2020-10-17), XP093172571, GB ISSN: 0026-8976, DOI: 10.1080/00268976.2020.1788189 Retrieved from the Internet: URL:https://www.zora.uzh.ch/id/eprint/1985 36/1/molphys.pdf> * abstract; figure 1 * * 3. Merthod * | 1,6,11 | |
| A | AVERY CHRIS ET AL: "Protein Function Analysis through Machine Learning", BIOMOLECULES, vol. 12, no. 9, 1 September 2022 (2022-09-01), page 1246, XP093172577, CH ISSN: 2218-273X, DOI: 10.3390/biom12091246 * abstract * * 3.4. Protein Dynamics * | 1,6,11 | |

**TECHNICAL FIELDS SEARCHED (IPC)**

G06N
G06F
G16B

The present search report has been drawn up for all claims

| Place of search | Date of completion of the search | Examiner |
|---|---|---|
| The Hague | 10 June 2024 | De Meyer, Arnaud |

EPO FORM 1503 03.82 (P04C01)

14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. RAY ; B. LINDSAY.** The topography of multivariate normal mixtures. *Annals of Statistics,* 2005, vol. 33, 2042-2065

- **HENNIG, C.** Ridgeline plot and clusterwise stability as tools for merging Gaussian mixture components. *Classification as a Tool for Research,* 2010, 109-116
- Bayes' theorem. **JAMES JOYCE.** The Stanford Encyclopedia of Philosophy. 2003